# EUROPÄISCHE PATENTSCHRIFT

(11) **EP 0 428 030 B1**
(45) Veröffentlichungstag und Bekanntmachung des Hinweises auf die Patenterteilung: **12.10.1994**
(21) Anmeldenummer: 90121090.6
(22) Anmeldetag: 03.11.1990
(51) Int. Cl.: C07D 307/33

(54) **Verfahren zur Abtrennung von gamma-Butyrolacton aus Gemischen, die Bernsteinsäurediethylester enthalten**
Process for the separation of gamma-butyrolactone from mixtures containing diethyl succinate
Procédé pour la séparation de gamma-butyrolactone de mélanges contenant du succinate de diéthyle

(30) Priorität: 16.11.1989 DE 3938121
(43) Veröffentlichungstag der Anmeldung: 22.05.1991
(73) Patentinhaber: BASF Aktiengesellschaft, 67063 Ludwigshafen (DE)
(72) Erfinder: Fischer, Rolf, Dr., W-6900 Heidelberg (DE); Stops, Peter, W-6701 Altrip (DE); Brunner, Erwin, Dr., W-6700 Ludwigshafen (DE); Weigand, Rudolf, W-6835 Bruehl (DE)

(56) Entgegenhaltungen:
- EP-A- 0 255 400
- EP-A- 0 256 813

## Beschreibung

Diese Erfindung betrifft ein verfahren zur Abtrennung von γ-Butyrolacton aus einem Gemisch, das Ethanol, Tetrahydrofuran, Wasser, n-Butanol, 1,4-Butandiol, Bernsteinsäurediethylester und γ-Butyrolacton enthält, durch Destillation.

1,4-Butandiol, ein wichtiges Zwischenprodukt für die Herstellung von Polyestern, wird z.B. durch Hydrierung der Diethylester der Maleinsäure, Fumarsäure oder Bernsteinsäure hergestellt. Bei diesem Verfahren fällt ein Gemisch an, das 1,4-Butandiol (BD) neben γ-Butyrolacton (GBL), Tetrahydrofuran (THF), Bernsteinsäurediethylester (BSDE), Ethanol, n-Butanol, Wasser und untergeordneten Mengen an hochsiedenden Nebenprodukten enthält.

Bei der Aufarbeitung dieser Gemische ist es erstrebenswert, nicht nur das als Hauptprodukt entstehende BD, sondern auch die weiteren Wertprodukte THF und GBL in reiner Form zu gewinnen.

Die Reindarstellung von GBL aus den genannten Gemischen ist durch einfache Destillation nicht möglich, da GBL mit BSDE ein Azeotrop bildet. In der EP-A 256 813 wird deshalb vorgeschlagen, den BSDE aus solchen Gemischen mit GBL durch Extraktion mit einem organischen Lösungsmittel abzutrennen. Nach einer bevorzugten Arbeitsweise wird der Extrakt, der aus BSDE und dem organischen Lösungsmittel besteht, nachträglich mit einem polaren Lösungsmittel, wie Wasser, extrahiert. Dieses Trennverfahren ist aufwendig, da zwei zusätzliche Stoffe benötigt werden und die nachträgliche destillative Abtrennung von Wasser hohe Energiekosten verursacht.

In der EP-A 255 400 wird ein Verfahren zur Reindarstellung von GBL beschrieben, bei dem die durch das Azeotrop aus GBL und BSDE bedingten Schwierigkeiten durch Zusatz von Maleinsäurediethylester umgangen werden. Die bei der katalytischen Hydrierung von Maleinsäure-, Fumarsäure- und Bernsteinsäurediethylester erhaltenen Gemische aus BD, THF, GBL, BSDE, Ethanol, n-Butanol und Wasser werden in einer ersten Kolonne so destilliert, daß Ethanol, Wasser, THF und n-Butanol über Kopf abgetrennt werden und sich alle anderen Komponenten im Sumpf der Kolonne befinden. Das Sumpfprodukt der ersten Kolonne wird in einer zweiten Kolonne so destilliert, daß über Kopf das Azeotrop aus GBL und BSDE abgenommen wird und das BD als Sumpfprodukt zurückbleibt. Zur Reingewinnung von GBL wird das Kopfprodukt der zweiten Kolonne zusammen mit einer passenden Menge Maleinsäurediethylester, der das Einsatzprodukt für die katalytische Hydrierung darstellt, auf eine dritte Kolonne gegeben. Hierbei wird reines GBL über Kopf abdestilliert. Das aus Maleinsäurediethylester und BSDE bestehende Sumpfprodukt kann in die Hydrierstufe zurückgeleitet werden.

In mehreren weiteren Kolonnen muß das Kopfprodukt der ersten Kolonne so aufgetrennt werden, daß spezifikationsgerechtes THF gewonnen, für die Veresterung von Maleinsäuremonobutylester geeignetes Ethanol zurückgeführt und Wasser und n-Butanol ausgeschleust werden können. Weiterhin muß aus dem Sumpfprodukt der zweiten Kolonne durch Destillation spezifikationsgerechtes BD gewonnen werden. Dieses Verfahren erfordert somit einen hohen Trennaufwand und benötigt zahlreiche Destillationskolonnen.

Es wurde nun gefunden, daß man GBL aus einem Gemisch, das Ethanol, THF, Wasser, n-Butanol, BD, BSDE und GBL enthält, durch Destillation erheblich vorteilhafter dadurch abtrennen kann, daß man
a) in einer ersten Kolonne mit einer theoretischen Bodenzahl von 5 bis 25, die bei einem Kopfdruck von 50 bis 500 mbar und einer Kopftemperatur von 35 bis 50°C betrieben wird, THF, Wasser und bis zu 99 % des Ethanols über Kopf abtrennt,
b) das Sumpfprodukt der ersten Kolonne in eine zweite Kolonne, die eine theoretische Bodenzahl von 10 bis 30 aufweist, einspeist, und das bei einem Kopfdruck von 20 bis 200 mbar und einer Kopftemperatur von 45 bis 60°C erhaltene Kopfprodukt, das Ethanol, n-Butanol, GBL, BSDE und BD enthält, in eine dritte Kolonne einspeist,
c) das in der dritten Kolonne, die eine theoretische Bodenzahl von 40 bis 100 aufweist, bei einem Kopfdruck von 20 bis 200 mbar und einer Kopftemperatur von 50 bis 65°C erhaltene Kopfprodukt, das Ethanol, n-Butanol und GBL enthält, in eine vierte Kolonne einspeist, und
d) in der vierten Kolonne, die eine theoretische Bodenzahl von 10 bis 30 aufweist, bei einem Kopfdruck von 50 bis 500 mbar und einer Kopftemperatur von 45 bis 55°C Ethanol und n-Butanol über Kopf abdestilliert, und das reine GBL einem Seitenabzug der Kolonne entnimmt.

Die für das neue Verfahren eingesetzten, GBL enthaltenden Gemische erhält man z.B. bei der bekannten katalytischen Hydrierung von Ethylestern der Maleinsäure, Fumarsäure und/oder Bernsteinsäure. Sie haben z.B. die folgende Zusammensetzung: 40 bis 55 Gew.% Ethanol, 0,5 bis 10 Gew.% THF, 0,05 bis 2 Gew.% n-Butanol, 0,005 bis 4 Gew.% Wasser, 2 bis 10 Gew.% GBL, 20 bis 50 Gew.% BD, 2 bis 10 Gew.% BSDE und 0,1 bis 1 Gew.% Hochsieder.

Das neue Verfahren wird unter Verwendung von vier Vakuumkolonnen z.B. folgendermaßen durchgeführt:
In der ersten Kolonne, die eine theoretische Bodenzahl von 5 bis 25 aufweist, wird aus dem Gemisch THF, Wasser und bis zu 99 %, vorzugsweise 97 bis 99 Gew.% des im Gemisch enthaltenen Ethanols über Kopf abdestilliert. Die Temperaturen und Drücke am Kopf betragen z.B. 35 bis 50°C und 50 bis 500 mbar. Das Rücklaufverhältnis beträgt z.B. 0,2 bis 1. Der Sumpf der ersten Kolonne, der n-Butanol, GBL, BD, BSDE und restliches Ethanol sowie Hochsieder enthält, wird in die zweite Kolonne mit einer theoretischen Bodenzahl von 10 bis 30 eingespeist, in der Ethanol, n-Butanol, GBL, BSDE und ein Teil des BD, welches mit BSDE ein Azeotrop bildet, über Kopf abgetrennt werden. Die Temperaturen und Drücke am Kopf der zweiten Kolonne liegen bei 45 bis 60°C und 20 bis 200 mbar. Das Rücklaufverhältnis liegt z.B. bei 1 bis 10. Das Sumpfprodukt der zweiten Kolonne enthält die Hauptmenge des BD und die Hochsieder.

Das Kopfprodukt der zweiten Kolonne, das z.B. zu 40 bis 90, insbesondere 55 bis 60 Mol.% aus GBL, zu 10 bis 40, insbesondere 25 bis 31 Mol.% aus BSDE, zu 0,1 bis 10, insbesondere 1 bis 4 Mol.% aus BD, zu 1 bis 20, insbesondere 5 bis 10 Mol.% aus Ethanol und zu 0,1 bis 10, insbesondere 1 bis 3 Mol.% aus n-Butanol besteht, wird in die dritte Kolonne geleitet, die eine theoretische Bodenzahl von 40 bis 100 aufweist. Man erhält in dieser Kolonne bei einem Kopfdruck von 20 bis 200 mbar, einer Kopftemperatur von 50 bis 65°C und einem Rücklaufverhältnis von 0,5 bis 8 ein Kopfprodukt aus n-Butanol, GBL und dem restlichen Ethanol, welches man in die vierte Kolonne einführt. Das Sumpfprodukt der dritten Kolonne ist ein Gemisch aus BD und BSDE, das zweckmäßigerweise in die Hydrierung des Maleinsäurediethylesters zurückgegeben wird.

Aus dem Kopfprodukt der dritten Kolonne werden in der vierten Kolonne die Alkohole Ethanol und n-Butanol über Kopf abdestilliert und das reine GBL im Seitenabzug gewonnen. Die vierte Kolonne hat 10 bis 30 theoretische Böden. Die Temperaturen und Drücke am Kopf der vierten Kolonne liegen bei 45 bis 55°C bzw. 50 bis 500 mbar. Das Rücklaufverhältnis liegt z.B. bei 0,5 bis 3. Als Sumpfprodukt der vierten Kolonne erhält man ein Gemisch aus GBL und Hochsiedern, die sich während der Destillation gebildet haben. Es kann ausgeschleust oder in die zweite Kolonne zurückgeführt werden.

Nach dem Verfahren dieser Erfindung läßt sich GBL aus den Ausgangsgemischen mit einem erheblich verringerten apparativen Aufwand abtrennen. Es können auch während der verschiedenen Destillationsschritte, z.B. durch Umesterung oder andere Reaktionen entstandene Leicht- oder Hochsieder, gegebenenfalls in der vierten Kolonne von GBL abgetrennt werden. Die Kopfprodukte der ersten und vierten Kolonne und das Sumpfprodukt der zweiten Kolonne können in an sich bekannter Weise weiter gereinigt werden, um spezifikationsgerechtes THF und BD und in die Veresterungsstufe rückführbares Ethanol zu gewinnen.

Da zu erwarten war, daß die Anwesenheit von Ethanol und n-Butanol in Gemischen mit GBL und BSDE die destillative Trennung von GBL und BSDE unmöglich machen würde, muß das vorteilhafte Ergebnis des erfindungsgemäßen Verfahrens als überraschend bezeichnet werden.

### Beispiel

Es wurde ein durch katalytische Hydrierung von Maleinsäurediethylester erhaltenes Reaktionsgemisch destilliert, das die folgende Zusammensetzung aufwies: 48,6 % Ethanol, 40,8 % BD, 4,3 % GBL, 4,3 % BSDE, 1,6 % THF, 0,1 % n-Butanol, 0,1 % Wasser und 0,2 % Hochsieder.

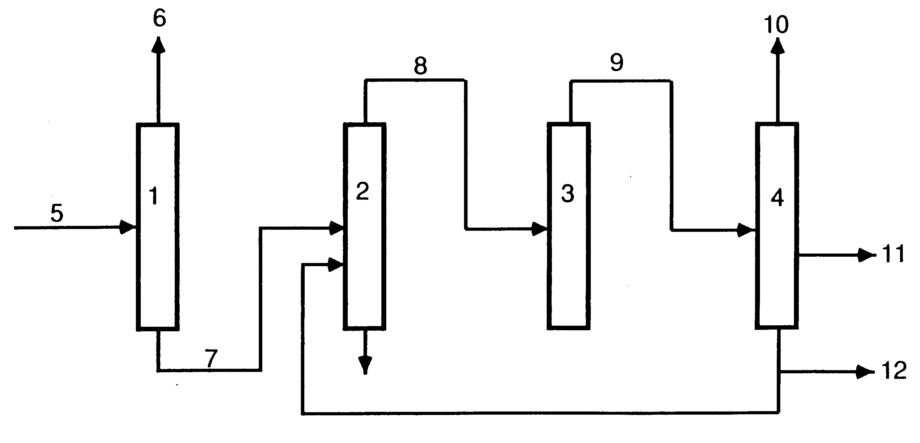

Das Schema der verwendeten Destillationsvorrichtung ist aus der Zeichnung ersichtlich. Über die Zuleitung (5) werden 100 Teile des Gemisches in die erste Kolonne (1) eingeleitet. Die Kolonne hat 15 theoretische Böden. Bei einer Kopftemperatur von 41°C und einem Kopfdruck von 200 mbar erhält man bei einem Rücklaufverhältnis von 0,3 500 Teile eines Kopfproduktes (6) mit der Zusammensetzung 96,5 % Ethanol, 3,3 % THF und 0,2 % Wasser. Als Sumpfprodukt werden 500 Teile eines Gemisches aus 81,6 % BD, 8,6 % BSDE, 0,6 % Ethanol, 0,2 % n-Butanol und 0,4 % Hochsiedern erhalten.

100 Teile Sumpfprodukt (7) aus der ersten Kolonne (1) werden in die zweite Kolonne (2) mit 15 theoretischen Böden geleitet. Bei einer Kopftemperatur von 54°C, einem Kopfdruck von 50 mbar und einem Rücklaufverhältnis von 1,7 erhält man 18,4 Teile Kopfprodukt der Zusammensetzung 46,9 % GBL, 46,9 % BSDE, 0,9 % n-Butanol und 2 % BD und 81,6 Teile Sumpfprodukt mit der Zusammensetzung 99,6 % BD und 0,4 % Hochsieder.

100 Teile Kopfprodukt (8) aus der zweiten Kolonne (2) werden in die dritte Kolonne (3) mit 60 Böden geleitet. Bei einer Kopftemperatur von 57°C, einem Kopfdruck von 50 mbar und einem Rücklaufverhältnis von 1,9 erhält man 51,3 Teile Kopfprodukt der Zusammensetzung 91,9 % GBL, 1,7 % n-Butanol und 6,4 % Ethanol und 48,7 Teile Sumpfprodukt mit der Zusammensetzung 95,5 % BD und 4,1 % BSDE.

100 Teile Kopfprodukt (9) der dritten Kolonne (3) werden in die vierte Kolonne (4) mit 15 theoretischen Böden geleitet. Bei einer Kopftemperatur von 51°C, einem Kopfdruck von 280 mbar und einem Rücklaufverhältnis von 0,8 erhält man 8,1 Teile Kopfprodukt (10) der Zusammensetzung 79,2 % Ethanol und 20,8 % n-Butanol. Durch den Seitenabzug (11) entnimmt man 90,6 Teile GBL in einer Reinheit von mindestens 99,9 %. Das Sumpfprodukt (12), das aus 1,3 Teilen GBL (Reinheit 99,9 %) und Spuren Hochsieder besteht, wird in die zweite Kolonne (2) zurückgeleitet.

## Patentansprüche

1. Verfahren zur Abtrennung von γ-Butyrolacton aus einem Gemisch, das Ethanol, Tetrahydrofuran, Wasser, n-Butanol, 1,4-Butandiol, Bernsteinsäurediethylester und γ-Butyrolacton enthält, durch Destillation, dadurch gekennzeichnet, daß man
a) in einer ersten Kolonne mit einer theoretischen Bodenzahl von 5 bis 25, die bei einem Kopfdruck von 50 bis 500 mbar und einer Kopftemperatur von 35 bis 50°C betrieben wird, Tetrahydrofuran, Wasser und bis zu 90 % des Ethanols über Kopf abtrennt,
b) das Sumpfprodukt der ersten Kolonne in eine zweite Kolonne, die eine theoretische Bodenzahl von 10 bis 30 aufweist, einspeist, und das bei einem Kopfdruck von 20 bis 200 mbar und einer Kopftemperatur von 45 bis 60°C erhaltene Kopfprodukt, das Ethanol, n-Butanol, γ-Butyrolacton, Bernsteinsäuredimethylester und 1,4-Butandiol enthält, in eine dritte Kolonne einspeist,
c) das in der dritten Kolonne, die eine theoretische Bodenzahl von 40 bis 100 aufweist, bei einem Kopfdruck von 20 bis 200 mbar und einer Kopftemperatur von 50 bis 65°C erhaltene Kopfprodukt, das Ethanol, n-Butanol und γ-Butyrolacton enthält, in eine vierte Kolonne einspeist, und
d) in der vierten Kolonne, die eine theoretische Bodenzahl von 10 bis 30 aufweist, bei einem Kopfdruck von 50 bis 500 mbar und einer Kopftemperatur von 45 bis 55°C Ethanol und n-Butanol über Kopf abdestilliert, und das reine γ-Butyrolacton einem Seitenabzug der Kolonne entnimmt.

## Claims

1. A process for separating γ-butyrolactone from a mixture which contains ethanol, tetrahydrofuran, water, n-butanol, 1,4-butanediol, diethyl succinate and γ-butyrolactone by distillation, wherein
a) in a first column which has from 5 to 25 theoretical plates and is operated at a top pressure of from 50 to 500 mbar and a top temperature of from 35 to 50°C, tetrahydrofuran, water and up to 90% of the ethanol are separated off at the top,
b) the bottom product of the first column is fed into a second column which has from 10 to 30 theoretical plates, and the top product which is obtained at a top pressure of from 20 to 200 mbar and a top temperature of from 45 to 60°C and contains ethanol, n-butanol, γ-butyrolactone, diethyl succinate and 1,4-butanediol is fed into a third column,
c) the top product which contains ethanol, n-butanol and γ-butyrolactone and is obtained in the third column, which has from 40 to 100 theoretical plates, at a top pressure of from 20 to 200 mbar and a top temperature of from 50 to 65°C is fed into a fourth column, and
d) in the fourth column which has from 10 to 30 theoretical plates, ethanol and n-butanol are distilled off at the top at a top pressure of from 50 to 500 mbar and a top temperature of from 45 to 55°C, and the pure γ-butyrolactone is taken off from the column as a side stream.

## Revendications

1. Procédé de séparation de la γ-butyrolactone d'avec un mélange qui contient de l'éthanol, du tétrahydrofuranne, de l'eau, du n-butanol, du 1,4-butanediol, du succinate de diéthyle et de la γ-butyrolactone, par distillation, caractérisé en ce que
a) on sépare en tête d'une première colonne d'un nombre de plateaux théorique de 5 à 25, à une pression de tête de colonne de 50 à 500 mbars et à une température de tête de colonne de 35 à 50°C, le tétrahydrofuranne, l'eau et jusqu'à 90% de l'éthanol,
b) on injecte le produit de fond de la première colonne dans une seconde colonne, qui présente un nombre de plateaux théorique de 10 à 30 et on injecte le produit de tête obtenu à une pression de tête de 20 à 200 mbars et une température de tête de 45 à 60°C, qui contient de l'éthanol, du n-butanol, de la γ-butyrolactone, du succinate de diméthyle et du 1,4-butanediol, dans une troisième colonne,
c) on injecte le produit de tête obtenu dans la troisième colonne, qui présente un nombre de plateaux théorique de 40 à 100, à une pression de tête de 20 à 200 mbars et à une température de tête de 50 à 65°C, qui contient de l'éthanol, du n-butanol et de la γ-butyrolactone, dans une quatrième colonne, et
d) on élimine par distillation en tête de la quatrième colonne, qui présente un nombre de plateaux théorique de 10 à 30, à une pression de tête de 50 à 500 mbars et une température de tête de 45 à 55°C, l'éthanol et le n-butanol et on prélève la γ-butyrolactone pure par une conduite d'évacuation latérale de la colonne.
